# EUROPEAN PATENT APPLICATION

(11) **EP 1 027 862 A1**
(43) Date of publication of application: **16.08.2000**
(21) Application number: 00101937.1
(22) Date of filing: 01.02.2000
(51) Int. Cl.: A61B 5/055, G01R 33/28

(54) **Apparatus for magnetic resonance tomograph**

(30) Priority: 10.02.1999 IT GE990015
(71) Applicant: PARAMED S.r.l., 17025 Loano, Province of Savona (IT)
(72) Inventor: Dutto, Roberto, 16127 Genova (IT); Monaldi, Marco, 16127 Genova (IT); Scarnati Felice, 16159 Genova (IT)
(74) Representative: Porsia, Attilio, Dr.

(57) **Abstract**

Apparatus for magnetic resonance imaging including a scanner (1, 101) and a support (16) for the patient. In said apparatus the scanner (1, 101) is movable with respect to the examination room and the patient support (16) is fixed or movable with respect to the scanner (1, 101) and/or the examination room.

## Description

The present invention refers to an apparatus for magnetic resonance imaging used in the medical field for the study of anatomic regions or parts thereof in patients subjected to said imaging technique.

The known magnetic resonance imaging scanners used for diagnostic purposes are usually equipped with big and heavy magnets generating an internal static magnetic field, necessary to generate the tomographic image, and an unwanted fringe magnetic field invading the surrounding space.

The safety provisions in force compel to check the area where the magnetic field exceeds the 5 Gauss limit, which is normally kept in the same room where the magnet is, said scanners requiring normally an area of 30 sq. meters. Recently have appeared on the market new systems for magnetic resonance imaging, equipped with a very small size magnet, which are optimized for the study of specific parts of the body: for this reason they are said "dedicated" systems. A consequence of their unique magnet features is a remarkable reduction in the space necessary for their installation.

In these last more recent cases, thanks to the remarkable reduction of the fringe magnetic field, the effective area occupied by the system depend not only on the presence of the magnet, but also on the exam position the patient is placed in with respect to the magnet.

As for the systems equipped with whole body magnet, characterized besides other things by high weight and wide fringe magnetic field, said magnet has always the same position with respect to the examination room and it is the patient, who is positioned according to the anatomic part to study, by means of suitable supports, such as for instance patient tables and/or seats.

Moreover, for economical and complexity reasons, even for the newest "dedicated" systems it has been maintained the same structure with the magnet fixed with respect to the examination room and the patient is correctly placed into the magnet rotating or translating said supports, such as patient tables and/or seats, around the magnet.

Aim of the present invention is the development of an apparatus for magnetic resonance imaging that includes a moving system, so that the complete apparatus composed of the moving system and the magnetic resonance unit is characterized by compactness, use flexibility and limited installation costs; moreover the installation area, substantially reduced with respect to the area required for the known systems, allow said apparatus to be used, for example, even in very small medical practices.

Object of the present invention is then an apparatus for magnetic resonance imaging including a scanner and a support for the patient; in said apparatus the scanner can be moved with respect to the examination room and the patient support can be fixed or moved with respect to the scanner and/or the examination room.

According to the present invention, the scanner can be either based on a close bore magnet (it is so said a magnet having at least two opposite openings such as the region to be examined has to be placed in a tunnel inside the magnet) or on an open bore magnet (it is so said a magnet open on three or more sides so improving the patient comfort, in particular in reference to possible claustrophobic reactions) and it is placed on the top of a supporting bench, provided with special supports for the rotation of the magnet on the bench top with respect to its axis and/or special supports for the translation of the magnet to any direction on said bench top.

According to a further feature of the present invention, the special supports for the scanner positioning and rotation are supported in their turn by the special supports for the translation provided in the bench, so allowing the magnet combined rotating and translating movements.

The present invention can be better understood from the following description to be considered as not limitative, and referred to the enclosed drawings where:
- Fig. 1 is a side view of a device for magnetic resonance imaging including a close bore magnet mechanically moved according to the present invention;
- Fig. 2 is a front view of the device of fig. 1 with the moving system in section;
- Fig. 3 is a side view of a device for magnetic resonance imaging including an open bore magnet mechanically moved according to the present invention;
- Fig. 4a, 4b, 4c show schematically a known scanner with the patient placed in three different positions with respect to the close bore magnet;
- Fig. 5a, 5b, 5c show schematically a scanner according to the present invention with the close bore magnet placed in three different positions with respect to the patient;
- Fig. 6a, 6b, 6c, 6d show schematically a known scanner with the patient placed in four different positions with respect to the open bore magnet;
- Fig. 7a, 7b, 7c, 7d show schematically a scanner according to the present invention with the patient placed in four different positions with respect to the open bore magnet;

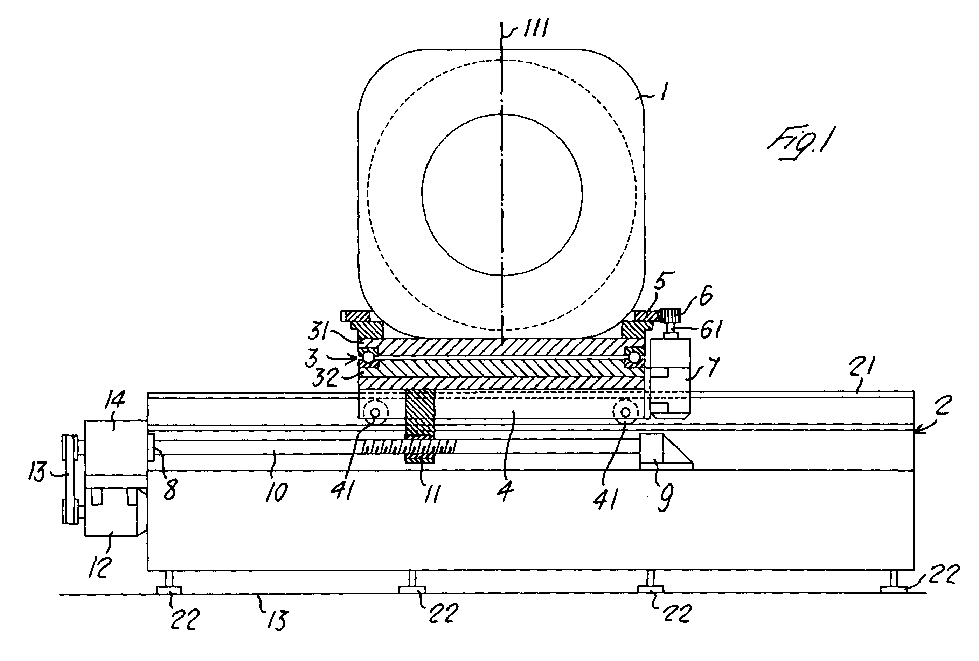

In Fig. 1 is shown a magnetic resonance imaging scanner based on a close bore magnet 1 having toroidal shape, supported by a fixed bench 2 which is placed, through the feet 22, on the floor 13 of an examination room, where the patient is subjected to the study of a certain anatomic part, such as for instance an upper or lower limb. The magnet 1 is supported by a bearing disc 3 composed of a lower plate 32 integral with the trolley 4, and an upper plate 31 that can rotate on said lower plate 32 around its axis, which in figure coincides with the axis 111 of the magnet 1. The magnet 1 is placed on the upper plate 31 and moreover a crown gear 5 is fastened to the same plate 31, engaged with its pinion 6 positioned by the side of the magnet 1. The pinion 6 is connected to an engine 7 through the shaft 61, where the engine 7 is provided to rotate said pinion so transmitting the rotation to the crown gear 5 and then to the plate 31 integral with it. The magnet 1 can then rotate through the gear "pinion 6 - crown gear 5" around its axis 111. The supporting top of the bearing disc 3, as mentioned before, is composed of the upper surface of the trolley 4 which, through the wheels 41, is free to translate horizontally to the right or to the left along the guides 21, obtained in the bench 2 between the two limit stops 8 and 9. Said limit stops 8 and 9 accommodate the support and rotation bearings of an endless screw 10 inserted into a nut screw 11, fastened to the trolley 4. The endless screw 10 is set into rotation by an engine 12 to which is connected a reduction unit 14, through a driving belt 13. As it can be noticed, the stroke of the trolley 4 is limited on the left and on the right by the contact of the nut screw 11 with the limit stops 8 and 9 respectively. Then, according to the present invention, the close bore magnet 1, shown in figure, is free to perform translating, rotating, and combinations of translating and rotating movements, with respect to the bench 2 on which it is placed.

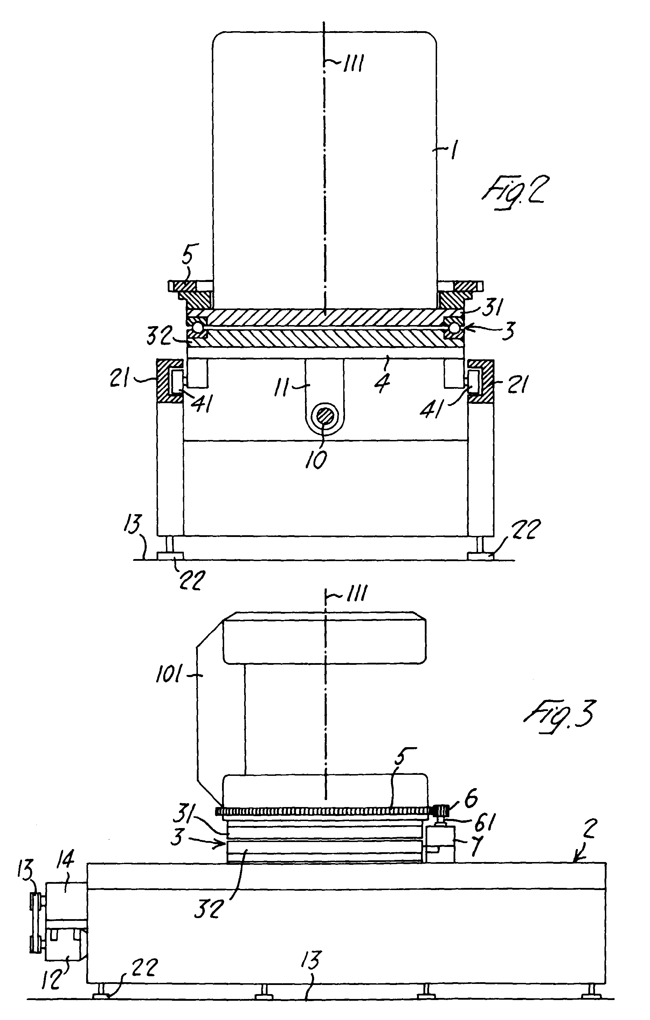

Fig. 2 is the front view of the device in Fig. 1, where it can be better seen the position of the guides 21 for the wheels 41 of the trolley 4 which, as said before, are obtained in the bench 2, and the position of the system "endless screw 10 - nut screw 11" that allows the trolley to slide along said guides. As the figure brings out, the moving system for the magnet 1 guarantees to the whole apparatus a great compactness and then a very limited area required for its installation.

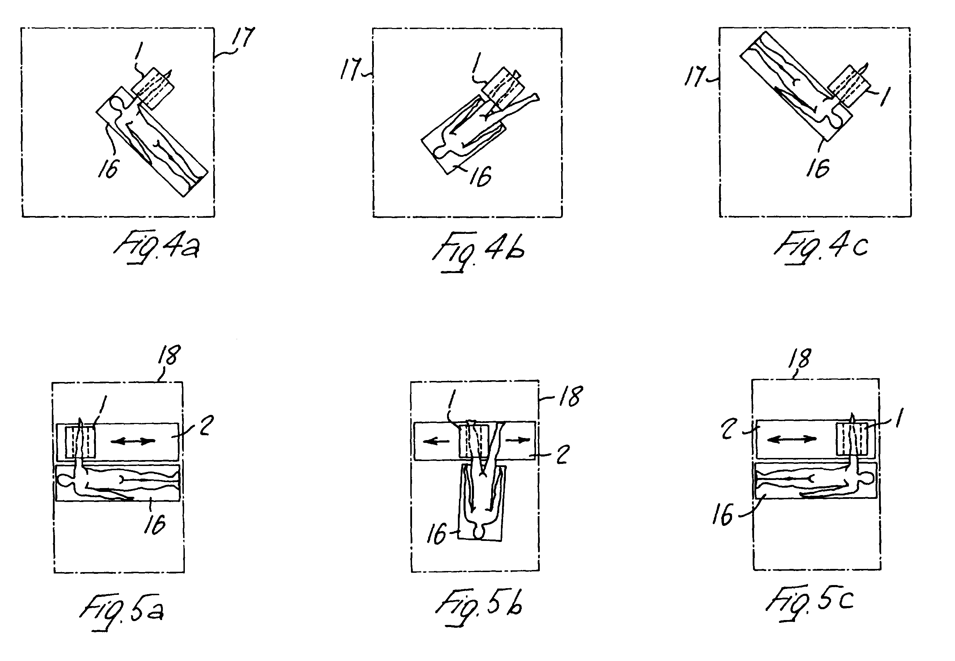

In Fig. 3 is shown a scanner including an open bore magnet 101, in the specific case a C shaped magnet is shown as an example, where the magnetic plates are placed over and under the patient's anatomic region to be studied, and not surrounding him completely as in the case of the close bore magnet. It is to notice that the supporting bench 2 for the magnet 101 is quite the same as the one described with references to Figs. 1 and 2, and so said magnet, even in this case, can rotate, translate, or rotate and translate, with respect to the bench 2 that is fixed to the floor 13 of the examination room.

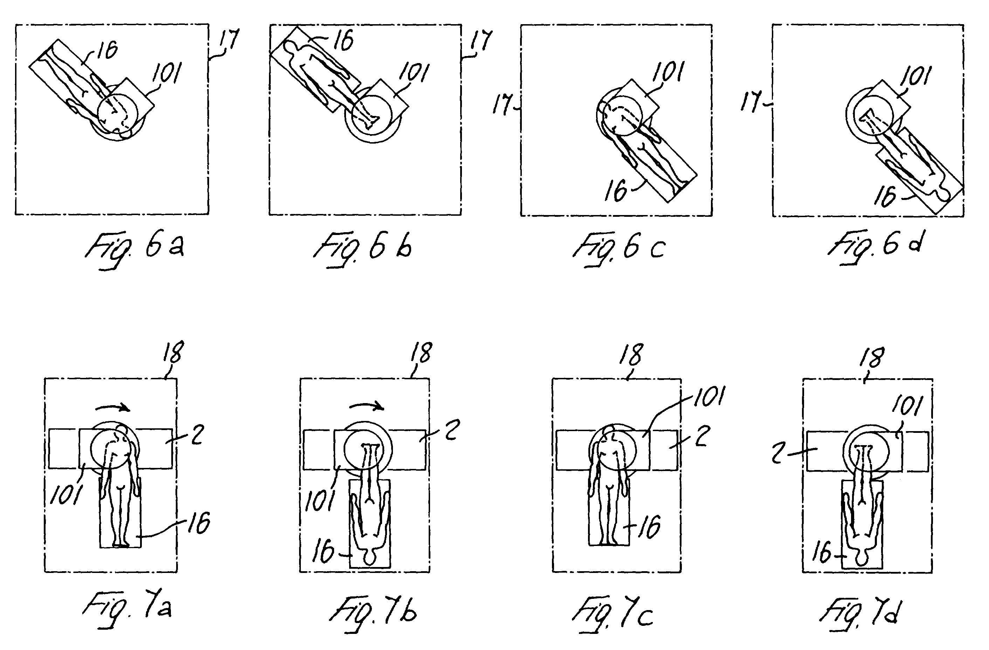

The relative movement of the magnet with respect to the supporting bench, as said up to this point, allows then to eliminate or limit the movements of the patient within the examination room, and then of the seat or table on which the patient lays. That remarkably reduces the effective area occupied by the scanner within the examination room, as it becomes clear by comparing Figs. 4a, b, c and Figs. 5a, b, c, which show respectively a known scanner and a scanner according to the present invention.

Looking at the Figs. 4a, b, c it becomes clear how the close bore magnet 1 is fixed with respect to the position taken by the patient laying on the table 16: as an example, it has been shown in Figs. 4a, b, c the situations the patient is subjected to for the examination of the left upper limb, the left lower limb and the right upper limb respectively. The necessary area for the movement of the table 16 is shown by the dotted line 17, outlining substantially the effective area required for the scanner within the examination room.

Considering now Figs. 5a, b, c, the scanner according to the present invention includes a magnet 1 supported by the bench 2 and the table 16 where the patient lays; from these figures it becomes clear that the movement of the magnet 1 requires a smaller area for positioning the patient with respect to the previous example, shown in figs. 4a, b, c. In particular it was meant to show three fixed exam positions, it is to say: exam of left upper limb (Fig. 5a), left lower limb (Fig. 5b), and fight upper limb (Fig. 5c). As it becomes clear from the combination of the horizontal translation movement of the magnet 1 and the patient table 16, it is possible to remarkably reduce the required area 18 with respect to the previously shown area 17. The stroke of the magnet 1 on the bench 2 can be for example 800 mm while the installation area can be reduced for example from 9 sq. m (3x3 m) minimum for the known scanner to about 6 sq. m (2x3) for the apparatus of the present invention, so saving about one third space.

In Figs. 6a, b, c, d is shown a known magnetic resonance imaging scanner with open bore magnet 101 placed in the middle of the area 17, where the table 16 is moved according to the examination the patient is subjected to, in particular it was meant to show four different exam positions, it is to say: exam of right upper limb (Fig. 6a), left lower limb (Fig. 6b), left upper limb (Fig. 6c) and right lower limb (Fig. 6d).

In Fig. 7a, b, c, d is shown a magnetic resonance imaging scanner according to the present invention, including an open bore magnet 101 supported by the bench 2, being the magnet 101 movable with respect to said bench, and a patient table 16. It is immediately noticeable that the area 18 required to submit the patient to the examinations mentioned for Figs. 6a, b, c, d, is remarkably reduced with respect to the required area 17. In particular is here shown the case where the magnet 101 is rotated 180° around its axis to the right looking at the figures, to move from the position taken in Figs. 7a, b (examination of right upper limb and right lower limb) to the position taken in Figs. 7c, d (examination of left upper limb and left lower limb). Translating, and rotating and translating movements of the magnet 101 along the bench 2 are naturally possible as well, and even in this case, with the apparatus according to the invention provided with open bore magnet, it is possible to reduce the encumbrance area to for example one third of the area necessary for the known technique scanners.

As noticed in the previous description the advantages achieved by using an apparatus for magnetic resonance imaging according to the embodiment form shown in the present document and claimed as a not limitative description, are numerous, and several are the possible development solutions to obtain said advantages without getting out of the field of the enclosed claims.

## Claims

1. An apparatus for magnetic resonance imaging including a scanner (1, 101) and a support (16) for the patient, characterized in that the scanner (1, 101) is movable with respect to the examination room and the patient support (16) is fixed or movable with respect to the scanner (1,101) and/or the examination room.

2. An apparatus for magnetic resonance imaging according to claim 1, characterized in that the scanner (1, 101) is positioned on the top of a supporting bench (2) and is movable with respect to said bench.

3. An apparatus for magnetic resonance imaging according to claim 1, characterized in that said scanner (1, 101) is based on a close bore magnet (1).

4. An apparatus for magnetic resonance imaging according to claim 1, characterized in that said scanner (1, 101) is based on an open bore magnet (101).

5. An apparatus for magnetic resonance imaging according to any of the previous claims, characterized in that the scanner (1, 101) is positioned on said bench (2) through supports (3, 31, 32) allowing its rotation on the bench (2) top with respect to its axis (111).

6. An apparatus for magnetic resonance imaging according to any of the previous claims, characterized in that the scanner (1, 101) is positioned on said bench (2) through supports (4, 21) allowing its translation on the bench (2) top.

7. An apparatus for magnetic resonance imaging according to any of the previous claims, characterized in that said positioning and rotation supports (3, 31, 32) of the scanner (1, 101) are supported by said translation supports (4, 21) provided in the bench (2), so allowing the scanner (1, 101) combined translating and rotating movements.

8. An apparatus for magnetic resonance imaging according to claims 5 and 7, characterized in that said rotation supports (3, 31, 32) are a bearing disc (3) provided with an upper rotating plate (31) on which is supported the scanner (1, 101), and a lower plate (32) fixed as to said upper plate (31), being said upper plate rotating thanks to suitable moving supports (5, 6, 7).

9. An apparatus for magnetic resonance imaging according to claim 8, characterized in that said moving supports (5, 6, 7) are a crown gear (5) integral with said upper plate (31) and set into rotation by means of a pinion (6) connected to moving supports (7).

10. An apparatus for magnetic resonance imaging according to claims 5 and 7, characterized in that said translation supports (4, 21) are a trolley (4) sliding through suitable moving supports (10, 11, 12, 13, 14) on substantially horizontal guides (21) obtained along the side surfaces of the bench (2).

11. An apparatus for magnetic resonance imaging according to claim 9, characterized in that said moving supports are an endless screw (10) set into rotation by means of suitable moving supports (12, 13, 14) and a nut screw (11) fastened to the trolley (4).

12. An apparatus for magnetic resonance imaging according to claim 9, characterized in that the stroke of the trolley (4) during the horizontal translation is limited by suitable limit stops (8, 9).
